# EUROPEAN PATENT APPLICATION

(11) **EP 4 621 432 A1**
(43) Date of publication of application: **24.09.2025**
(21) Application number: 24275031.3
(22) Date of filing: 22.03.2024
(51) Int. Cl.: G01R 33/3815

(54) **MAGNETIC RESONANCE DEVICE WITH SHORT PATIENT BORE**

(71) Applicant: Siemens Healthcare Limited, Camberley, Surrey GU15 3YL (GB)
(72) Inventor: Mallett, Michael, Faringdon, SN7 8EL (GB)
(74) Representative: Siemens Healthineers Patent Attorneys

(57) **Abstract**

The invention relates to a magnet arrangement (11) for a magnetic resonance imaging device (10), comprising a main magnet (12) including a plurality of superconducting coils (31), a reversed superconducting coil (32), and a ferromagnetic element (33), wherein the reversed superconducting coil (32) is arranged between two superconducting coils (31) of the plurality of superconducting coils (31), and wherein the ferromagnetic element (33) is arranged between the reversed superconducting coil (32) and a superconducting coil (31).

## Description

Independent of the grammatical term usage, individuals with male, female or other gender identities are included within the term.

Conventional magnetic resonance devices for medical diagnostics typically weigh several tons and have a footprint of at least three square meters. Such devices can only be installed in places that meet special requirements regarding accessibility, but also load-bearing capacities of floors or ceilings. For example, an examination room accommodating the magnetic resonance device must have a sufficiently high ceiling and appropriately designed access paths. Furthermore, the floor of the examination room must be able to withstand the weight of the magnetic resonance device over a long period of time.

Smaller medical institutions and practices interested in new magnetic resonance imaging applications (such as dentistry, neurology, orthopaedics) are often not equipped for transporting and installing conventional magnetic resonance devices. For example, installing a conventional magnetic resonance device in a dental practice may be difficult, as customers may not accept an interruption in operations due to construction sites or renovation work such as opening walls or enlarging doors.

Furthermore, an adoption of new magnetic resonance imaging applications depends on the costs of a magnetic resonance device, which are strongly influenced by the length of a patient bore enclosed by a main magnet of the magnetic resonance device. Assuming a predetermined or constant homogeneity volume, the main magnet of a magnetic resonance device typically becomes more expensive with a decrease in length of the patient bore. Shorter magnets may also require customized support structures configured to endure unconventional distributions of (electromagnetic) forces which can result from the shortening of the patient bore.

It is an objective of the invention to mitigate disadvantages arising from decreasing a length of a patient bore in a magnetic resonance device.

This objective is achieved by a magnet arrangement and a magnetic resonance device according to the invention. Further advantageous embodiments are specified in the dependent claims.

The inventive magnet arrangement is configured to be used in a magnetic resonance imaging device. The magnet arrangement may be configured to provide a main magnetic field suitable for magnetic resonance imaging, particularly diagnostic medical imaging, of an object.

According to the invention, the magnet arrangement comprises a main magnet including a plurality of superconducting coils, a reversed superconducting coil, and a ferromagnetic element.

The magnet arrangement may comprise two sets of superconducting coils. Particularly, the magnet arrangement may comprise a set of "inner coils" and a set of "outer coils".

The "inner coils" of the magnet arrangement may form the main magnet or constitute a part of the main magnet. The "inner coils" may be arranged sequentially along a common axis, particularly a cylinder axis or an axis of rotational symmetry of the main magnet. It is conceivable that the "inner coils" are arranged coaxially along the common axis and/or comprise an approximately common radius.

The main magnet may comprise a plurality of superconducting coils. For example, the main magnet may comprise five to nine superconducting coils. In a preferred embodiment, the main magnet comprises five or six superconducting coils. The plurality of superconducting coils of the main magnet correspond to the "inner coils" of the magnet arrangement.

The set of "outer coils" (or "shielding coils") of the magnet arrangement can be arranged coaxially on a larger radius than the "inner coils". The "outer coils" may be configured to actively shield the "inner coils" of the main magnet from surrounding electromagnetic fields and/or electromagnetic radiation. According to an embodiment, the magnet arrangement comprises two "outer coils". However, the magnet arrangement may comprise a single "outer coil" or up to five "outer coils". The "outer coils" are not considered to form a part of the main magnet.

The reversed superconducting coil may form a part of the "inner coils" of the magnet arrangement. Particularly, the reversed superconducting coil may be arranged coaxially to the plurality of superconducting coils. For example, an axis of the reversed superconducting coil may correspond to a common axis defined by the plurality of superconducting coils. The common axis defined by the plurality of superconducting coils may correspond to a cylinder axis or an axis of rotational symmetry of the main magnet.

A reversed superconducting coil may be characterized by a reversed current direction with respect to the plurality of superconducting coils. Particularly, the reversed superconducting coil may be configured to provide a magnetic field which is oriented in an opposite direction with respect to the magnetic field provided by the plurality of superconducting coils. Apart from the current direction, a material and/or coil structure of the reversed superconducting coil may substantially correspond to a material and/or coil structure of the plurality of superconducting coils. For example, a dimension and/or a mass of superconducting wire of the reversed superconducting coil may substantially correspond to a dimension and/or a mass of a superconducting coil of the plurality of superconducting coils.

The reversed superconducting coil is arranged between two superconducting coils of the plurality of superconducting coils.

In a preferred embodiment, the reversed superconducting coil is mechanically connected to at least one superconducting coil of the plurality of superconducting coils. Particularly, the reversed superconducting coil and the plurality of superconducting coils of the main magnet may form a cohesive structure.

The reversed superconducting coil may be arranged or positioned between a first superconducting coil and a second superconducting coil. Preferably, the reversed superconducting coil is mechanically connected to the first superconducting coil and/or the second superconducting coil.

According to an embodiment, one or more spacers are arranged between the reversed superconducting coil and the first superconducting coil. It is also conceivable that one or more spacers are arranged between the reversed superconducting coil and the second superconducting coil. In a preferred embodiment, a spacer and/or a ferromagnetic element is arranged between the reversed superconducting coil and the first superconducting coil.

The reversed superconducting coil may be directly attached to the spacer, the ferromagnetic element and/or a superconducting coil via a form-locking connection, a force-locking connection and/or a material bond. For example, the reversed superconducting coil may be screwed, bolted and/or glued to the spacer, the ferromagnetic element and/or the superconducting coil.

It is conceivable that the reversed superconducting coil, the ferromagnetic element, and the plurality of superconducting coils are integrally bonded.

The main magnet may comprise a magnet support structure configured to provide mechanical support to the main magnet. The magnet support structure may be configured to maintain a predefined spatial arrangement of the reversed superconducting coil, the ferromagnetic element, and the plurality of superconducting coils. The magnet support structure may be configured to be mechanically connected to a support structure, particularly an outer vacuum chamber, of a magnetic resonance device.

The magnet arrangement may further comprise a cryogen vessel and/or a thermal shield. It is conceivable that the magnet arrangement is circumferentially enclosed in an outer vacuum chamber. The vacuum chamber may be formed as a double-walled hollow cylinder comprising an outer shell and an inner shell connected by annular end pieces. The magnet arrangement may be enclosed between the outer shell and the inner shell of the outer vacuum chamber. The inner shell of the outer vacuum chamber may correspond to a patient bore of a magnetic resonance device comprising the magnet arrangement.

In a conventional magnet arrangement without a reversed superconducting coil, inter-coil forces or electromagnetic forces acting on the superconducting coils of the main magnet are typically directed towards a central symmetry plane of the main magnet. Thus, the inter-coil forces in a conventional magnet arrangement are compressive in nature and tend to pull the superconducting coils towards the central symmetry plane.

For example, in a cylindrical magnet arrangement without a reversed superconducting coil, axial forces between the superconducting coils may be directed towards the central symmetry plane of the main magnet. Depending on a design of the main magnet, the axial forces may cause an equivalent weight of more than 10 tons or more than 100 tons to act on individual superconducting coils. Thus, superconducting coils arranged in proximity to the central symmetry plane may be subjected to significant compressional forces.

The reversed superconducting coil may be configured to modify the inter-coil forces within the main magnet in such a way that an electromagnetic force acting on at least one of the two superconducting coils framing the reversed superconducting coil is expansive rather than compressive (with respective to a central symmetry plane of the main magnet). Particularly, the reversed superconducting coil may be configured to reduce or invert an electromagnetic force acting on at least one superconducting coil, particularly an end coil, in comparison to a conventional magnet arrangement without a reversed superconducting coil.

The reversed superconducting coil may be configured in such a way to allow for a length of a patient bore to be decreased whilst retaining a desired dimension of a homogeneity volume provided via the main magnet. Particularly, the reversed superconducting coil may be configured to reduce a length of the main magnet below 1.3 m, below 1.2 m, below 1.1 m, or even below 1 m without compromising the homogeneity volume provided via the main magnet.

The homogenous volume may represent an imaging volume of a magnetic resonance device comprising the magnet arrangement. Particularly, the homogenous volume may correspond to a volume within a magnetic field provided by the main magnet. A uniformity of the magnetic field within the homogenous volume may exceed a predefined threshold.

The objective of decreasing the length of a patient bore may make the objective of providing a homogenous volume significantly more difficult. In introducing reversed superconducting coils, a number of variables can be introduced, e. g. by allowing a current direction to vary from a nominal "positive" to also a "negative" current direction, which favourably allows for finding a solution to both objectives.

From a different perspective, a summation of individual coil harmonics of superconducting coils moving closer together to reduce the length of the patient bore may not allow for the magnetic field homogeneity of the imaging volume to be within a desired range, because relative harmonic ratios from only "positive" superconducting coils may not produce a desirable solution. In introducing reversed superconducting coils providing inverted harmonics (e. g. by allowing for "negative" superconducting coils), a solution to the above-mentioned problems may be found.

According to the invention, the ferromagnetic element is arranged between the reversed superconducting coil and a superconducting coil.

For example, the ferromagnetic element may be arranged between the reversed superconducting coil and the first superconducting coil or between the reversed superconducting coil and the second superconducting coil.

The ferromagnetic element may be directly attached to the spacer, the reversed superconducting coil and/or a superconducting coil via a form-locking connection, a force-locking connection and/or a material bond.

The inventive magnet arrangement may comprise a plurality of reversed superconducting coils and a plurality of ferromagnetic elements. Particularly, the number of reversed superconducting coils may correspond to the number of ferromagnetic elements. In a preferred embodiment, the magnet arrangement comprises two reversed superconducting coils and two ferromagnetic elements. Each ferromagnetic element may be arranged adjacent to a reversed superconducting coil.

In introducing a reversed superconducting coil into a magnet arrangement, the number of design parameters affecting both the dimension of the main magnet and the dimension of the homogenous volume may favourably be increased. Particularly, the reversed superconducting coils may allow for shortening a patient bore of the main magnet without compromising a quality of a homogeneity volume provided via the main magnet.

In introducing a reversed superconducting coil into the main magnet, an absolute mass of superconducting wire in the main magnet may increase because the reversed superconducting coils may reduce a strength of the generated magnetic field which needs to be compensated by an increase in the size of the plurality of superconducting coils. Furthermore, the reversed superconducting coils may affect the inter-coil forces within the main magnet as described above. Thus, a dimension of the reversed superconducting coil may need to be restricted in such a way that the electromagnetic forces acting on the superconducting coil(s) can be adequately supported by a magnet support structure and the total axial forces on the conventional coils are compressive in nature.

In providing a magnet arrangement comprising a ferromagnetic element, a size limitation of the reversed coils may favourably be removed. Particularly, the ferromagnetic element may favourably restrain or even invert an expansive or outwardly directed electromagnetic force acting on a superconducting coil, especially an end coil, of the magnet arrangement.

For example, a ferromagnetic element may become magnetised by the magnetic field created by one or more superconducting coils. As a result of becoming magnetised, the ferromagnetic element may create a magnetic field opposing the magnetising field (i. e. the magnetic field created by the one or more superconducting coils). A summation of the magnetic fields of the one or more superconducting coils and the ferromagnetic element may result in a reduction of the overall magnetic field in a direction away from the one or more superconducting coils (or away from a majority of the superconducting coils if superconducting coils are arranged on both sides of a ferromagnetic element). Thus, the ferromagnetic element may act as a magnetic shield.

Thus, a requirement of the magnet support structure to be configured to resist expansive forces may favourably be omitted. Furthermore, the length of the main magnet, but also the patient bore, may favourably be reduced without increasing a dimension of the support structure.

According to an embodiment of the inventive magnet arrangement, the ferromagnetic element is arranged between the reversed superconducting coil and a superconducting coil, wherein the superconducting coil is an end coil of the main magnet.

An end coil may be arranged at an outer axial end of the main magnet. Particularly, the magnet arrangement may comprise two end coils framing or confining the ferromagnetic element, the reversed superconducting element, but also remaining superconducting coils, from two opposing directions. An end coil may be understood as a first superconducting coil or a starting superconducting coil of the main magnet. An end coil may also represent a final superconducting coil or a terminating superconducting coil of the main magnet. An end coil may terminate the main magnet in one direction.

In arranging the ferromagnetic element between a reversed superconducting coil and a superconducting coil, an electromagnetic interaction between the reversed superconducting coil and the superconducting coil may be reduced. Particularly, the ferromagnetic element may provide a level of magnetic shielding between the reversed superconducting coil and the superconducting coil. Thus, magnetic forces, particularly repulsive electromagnetic forces, between the superconducting coil and the reversed superconducting coil may be reduced, which may favourably facilitate a design and/or construction of the magnet support structure.

According to an embodiment of the inventive magnet arrangement, the ferromagnetic element is arranged directly adjacent to the reversed superconducting coil and/or the superconducting coil.

The ferromagnetic element may be sandwiched between the superconducting coil and the reversed superconducting coil. Particularly, the ferromagnetic element may be confined by the superconducting coil and the reversed superconducting coil in two opposing directions. For example, the superconducting coil may confine the ferromagnetic element in a first direction and the reversed superconducting coil may confine the ferromagnetic element in a second direction opposing the first direction.

The ferromagnetic element may be in direct mechanical contact with the superconducting coil and/or the reversed superconducting coil. Particularly, the ferromagnetic element may be mechanically connected to the superconducting coil and/or the reversed superconducting coil via a form-locking connection, a force-locking connection and/or a material bond.

In a further embodiment, the ferromagnetic element is arranged directly adjacent to a spacer. The spacer may be mechanically connected to the superconducting coil and/or the reversed superconducting coil.

A ferromagnetic element may favourably replace a spacer required for maintaining a predefined distance between the reversed superconducting coil and the superconducting coil. Furthermore, a ferromagnetic element directly attached to the reversed superconducting coil may favourably improve a structural integrity of the reversed superconducting coil, which may have less coil windings in comparison to the plurality of superconducting coils.

According to an embodiment of the inventive magnet arrangement, the main magnet comprises at least one spacer arranged between the ferromagnetic element and the superconducting coil and/or between the ferromagnetic element and the reversed superconducting coil.

It is conceivable that the ferromagnetic element is separated from the reversed superconducting coil and/or the superconducting coil via the at least one spacer.

Preferably, the at least one spacer is implemented as a ring, a plurality of rings, a hollow cylinder, a plurality of hollow cylinders, or a segment of a ring or a hollow cylinder. It is also conceivable that the at least one spacer is implemented as a block or a plurality of blocks of any suitable shape.

Preferably, the at least one spacer comprises or consists of a thermally conductive material. Particularly, the at least one spacer may be configured to transport thermal energy between the ferromagnetic element and the superconducting coil and/or the ferromagnetic element and the reversed superconducting coil. The at least one spacer may comprise an electrically insulating material, an electrically insulating coating, or an electrically insulating layer. According to an embodiment, the at least one spacer comprises a glass-reinforced-polymer (i. e. GRP or fibreglass) and a thermally conductive element, such as a piece of aluminium, copper or the like, attached or glued to the superconducting coil, the ferromagnetic element and/or the reversed superconducting coil to improve a transport of heat energy.

The at least one spacer may be configured to fill a gap between the ferromagnetic element and the superconducting coil, but also the ferromagnetic element and the reversed superconducting coil. The ferromagnetic element, the superconducting coil, the reversed superconducting coil and at the least one spacer may form a cohesive structure. The at least one spacer may be attached to the ferromagnetic element and the superconducting coil, but also the ferromagnetic element and the reversed superconducting coil, via a force-locking connection, a form-locking connection and/or a material bond.

In a preferred embodiment, the magnet arrangement comprises a first spacer arranged between the superconducting coil and the ferromagnetic element, and a second spacer arranged between the ferromagnetic element and the reversed superconducting coil. The superconducting coil, the first spacer, the ferromagnetic element, the second spacer and the reversed superconducting coil may be integrally bonded to form a cohesive structure.

A spacer may favourably allow for the superconducting coil and the reversed coil, but also other superconducting coils of the main magnet, to be arranged at predefined distances from one another. In spacing individual superconducting coils, the homogeneity of the imaging volume provided by the main magnet may favourably be improved.

A spacer may favourably be added if a dimension of the ferromagnetic element, particularly an axial length of the ferromagnetic element, is insufficient to provide a desired distance between the reversed superconducting coil and the superconducting coil.

Furthermore, the at least one spacer may comprise or consist of an electrically insulating material. Thus, the at least one spacer may favourably provide an electrical insulation between the ferromagnetic element and the superconducting coils of the main magnet (including the reversed superconducting coil).

In a preferred embodiment of the inventive magnet arrangement, the ferromagnetic element comprises the shape of a ring, a tube, a hollow cylinder, or a hollow prism.

The ferromagnetic element may comprise an annular, an oval, or a polygonal cross-section. Preferably, the ferromagnetic element comprises the shape of a hollow cylinder.

An inner diameter of the ferromagnetic element may correspond to an inner diameter of the superconducting coils and/or the reversed superconducting coil of the main magnet. However, the inner diameter of the ferromagnetic element may also exceed an inner diameter of the superconducting coils and/or the reversed superconducting coils. It is also conceivable that the inner diameter of at least one superconducting coil and/or at least one reversed superconducting coil of the main magnet exceeds the inner diameter of the ferromagnetic element.

According to an embodiment of the inventive magnet arrangement, the main magnet comprises a cylindrical shape, and the ferromagnetic element is arranged coaxially to the main magnet.

A cylinder axis or an axis of rotational symmetry of the ferromagnetic element may correspond to the cylinder axis or the axis of rotational symmetry of the main magnet.

A ferromagnetic element according to an embodiment described above may favourably allow for the patient bore to pass through the main magnet including the ferromagnetic element. In a preferred embodiment, the ferromagnetic element favourably prevents a restriction of a diameter of the patient bore.

According to an embodiment of the inventive magnet arrangement, a projection of an axial cross-sectional area of the superconducting coil along a cylinder axis of the main magnet and a cross-sectional area of the ferromagnetic element have a non-empty intersection.

According to an embodiment, a projection of an outer circumference of the superconducting coil along a cylinder axis of the main magnet intersects a section of the ferromagnetic element. For example, an inner diameter of the superconducting coil may exceed an inner diameter of the ferromagnetic element, whereas an outer diameter of the ferromagnetic element exceeds the inner diameter of the superconducting coil.

In a further embodiment, a projection of an inner circumference of the superconducting coil along the cylinder axis of the main magnet intersects a section of the ferromagnetic element. For example, an outer diameter of the ferromagnetic element may exceed an outer diameter of the superconducting coil, whereas the outer diameter of superconducting coil exceeds the inner diameter of the ferromagnetic element.

It is conceivable that the outer diameter of the ferromagnetic element substantially corresponds to an outer diameter of the reversed superconducting coil and/or the outer diameter of the superconducting coil. For example, an absolute deviation between the outer diameter of the ferromagnetic element and the outer diameter of the superconducting coil may amount to less than 5 % or less than 10 %. Likewise, the inner diameter of the ferromagnetic element may substantially correspond to an inner diameter of the reversed superconducting coil and/or the inner diameter of the superconducting coil.

The superconducting coil may correspond to an end coil according to an embodiment described above.

A ferromagnetic element according to the invention may favourably facilitate a mechanical connection and/or bonding between the superconducting coil and the ferromagnetic element, but also between the reversed superconducting coil and the ferromagnetic element. Particularly, a ferromagnetic element according to the invention may allow for an improved mechanical stability or a load-bearing capacity of the magnet arrangement.

In a preferred embodiment of the magnet arrangement, the ferromagnetic element comprises or consists of a high permeability material.

The ferromagnetic element may comprise sections consisting of a high permeability material. For example, the ferromagnetic element may comprise an inner cylinder and an outer cylinder. The inner cylinder may be configured to carry the outer cylinder and/or provide mechanical support to the outer cylinder. The inner cylinder may be mechanically coupled to the superconducting coil and/or the reversed superconducting coil. The outer cylinder may comprise or consist of the high permeability material.

It is also conceivable that sections consisting of high permeability material are distributed along an inner circumference or an outer circumference of the ferromagnetic element in regular or irregular intervals.

According to an embodiment, sections consisting of a high permeability material are embedded into a support structure of the ferromagnetic element. However, the ferromagnetic element may also consist of the high permeability material.

Examples of high permeability materials are metals, such as iron, cobalt, or nickel, but also alloys of these metals. For example, the ferromagnetic element may comprise or consist of an alloy of iron, cobalt and/or nickel. Preferably, the ferromagnetic element consists of iron or an iron alloy.

A ferromagnetic element according to the invention may favourably be magnetically saturated via the magnetic field of the main magnet and provide a magnetic flux link between the superconducting coil and the reversed superconducting coil. Thus, an increased magnetic field homogeneity within the imaging volume may be provided.

According to a further embodiment of the magnet arrangement, the ferromagnetic element is attached to the reversed superconducting coil and the superconducting coil.

The ferromagnetic element can be mechanically connected to the reversed superconducting coil and the superconducting coil according to an embodiment described above. Particularly, the ferromagnetic element can be attached to the reversed superconducting coil and the superconducting coil via a force-locking connection, a form-locking connection and/or a material bond. For example, the ferromagnetic element may be attached to the reversed superconducting coil and the superconducting coil via a screw connection, a bolt connection and/or an adhesive joint.

A surface of the ferromagnetic element may be in direct mechanical contact with a surface of the reversed superconducting coil and/or a surface of the superconducting coil. Particularly, a first axial face of the ferromagnetic element may be in direct mechanical contact with an axial face of the superconducting coil and a second axial face of the ferromagnetic element may be in direct mechanical contact with an axial face of the reversed superconducting coil. The first axial face and the second axial face of the ferromagnetic element may represent opposite sides, particularly opposite axial ends, of the ferromagnetic element.

In a preferred embodiment, the ferromagnetic element is attached to the reversed superconducting coil and the superconducting coil in such a way to form a coherent structure.

In attaching the ferromagnetic element to the reversed superconducting coil and the superconducting coil, a mechanical stability of the main magnet may favourably be improved.

According to an embodiment of the inventive magnet arrangement, the superconducting coil represents an end coil. The ferromagnetic element is configured to modify an inter-coil force within the main magnet in such a way that an outwardly directed force, which would act on the end coil of the main magnet in absence of the ferromagnetic element, is reduced by at least 30 %, at least 40 %, at least 50 %, or at least 60 %.

The main magnet may be configured in such a way that, in absence of the ferromagnetic element, an outwardly directed force or expansive force would act on the end coil. Particularly, the reversed superconducting coil may affect the inter-coil forces within the main magnet in such a way that the electromagnetic force acting on the end coil is directed away from the central symmetry plane of the main magnet.

The ferromagnetic element may be configured to modify the inter-coil forces between the end coil and the reversed superconducting coil in such a way, that the expansive electromagnetic force that would act on the end coil in absence of the ferromagnetic element is reduced by at least 30 %, at least 40 %, at least 50 %, or preferably at least 60 % by arranging the ferromagnetic element between the end coil and the reversed superconducting coil.

A ferromagnetic element configured to reduce an outwardly directed force acting on the end coil by at least 30 % or at least 50 % may exhibit a comparatively small axial dimension. Furthermore, the ferromagnetic element may reduce requirements for a magnet support structure containing the expansive forces. Thus, a weight and/or costs associated with the magnet arrangement may favourably be reduced or optimized.

According to a further embodiment, the ferromagnetic element is configured to modify the inter-coil force within the main magnet in such a way that an inwardly directed force is acting on the end coil.

As described above, the reverse superconducting coil and the end coil may be configured in such a way that, in absence of the ferromagnetic element, an outwardly directed force would act on the end coil.

The ferromagnetic element may be configured to modify the inter-coil forces between the end coil and the reversed superconducting coil in such a way that any expansive electromagnetic force acting on the end coil in absence of the ferromagnetic element is cancelled out or inverted by arranging the ferromagnetic element between the superconducting coil and the reversed superconducting coil.

A ferromagnetic element configured to cancel out or invert an outwardly directed force acting on an end coil may favourably allow for neglecting expansive electromagnetic forces when designing a support structure for the main magnet or magnet arrangement. Thus, a weight and/or costs associated with the support structure may favourably be decreased.

According to another embodiment of the inventive magnet arrangement, the superconducting coil represents an end coil. The ferromagnetic element is configured to modify an inter-coil force within the main magnet in such a way that an inwardly directed force, which would act on an end coil of the main magnet in absence of the ferromagnetic element, is increased by at least 10 %, at least 20 %, or at least 30 %.

The reversed superconducting coil and the end coil of the magnet arrangement may be configured in such a way that, in absence of the ferromagnetic element, an inwardly directed force would act on the end coil. Particularly, the reversed superconducting coil may affect the inter-coil forces within the main magnet in such a way, that the electromagnetic force acting on the end coil is compressive. However, the compressive force acting on the end coil may be less than a compressive force in a conventional magnet arrangement without a reversed superconducting coil.

The ferromagnetic element may be configured to modify inter-coil forces at an axial end of the main magnet in such a way, that a compressive electromagnetic force that would be acting on the end coil in absence of the ferromagnetic element is increased by arranging the ferromagnetic element between the end coil and the reversed superconducting coil.

A ferromagnetic element configured to increase an inwardly directed electromagnetic force acting on the end coil may favourably allow for a further reduction of weight and/or costs of a magnet support structure.

According to a further embodiment, the magnet arrangement comprises a first ferromagnetic element and a second ferromagnetic element.

The first ferromagnetic element and the second ferromagnetic element may correspond to an embodiment of the ferromagnetic element described above.

According to the invention, the first ferromagnetic element is arranged between a first reversed superconducting coil and a first superconducting coil, and the second ferromagnetic element is arranged between a second reversed superconducting coil and a second superconducting coil.

The first superconducting coil may correspond to an end coil arranged at a first end of the main magnet. The second superconducting coil may correspond to an end coil arranged at a second end of the main magnet. The first end and the second end of the main magnet may represent axial ends of the main magnet. Particularly, the first end and the second end of the main magnet may represent opposite ends of the main magnet.

According to an embodiment, the first superconducting coil, the first ferromagnetic element and the first reversed superconducting coil are arranged symmetrically to the second superconducting coil, the second ferromagnetic element and the second reversed superconducting coil with respect to the central symmetry plane of the main magnet.

It is also conceivable, that the first superconducting coil and the second superconducting coil represent bulk coils. For example, the first superconducting coil may be arranged at a predefined distance to a first end coil and the second superconducting coil may be arranged at a predefined distance to a second end coil.

In providing a magnet arrangement comprising at least two reversed superconducting coils and at least two ferromagnetic elements arranged symmetrically with respect to a central symmetry plane of the main magnet, the homogeneity of the magnetic field may favourably be improved in comparison to conventional magnet arrangements comprising a similar dimension .

The inventive magnetic resonance device is configured to acquire magnetic resonance data of an object positioned within an imaging region of the magnetic resonance device.

Preferably, the magnetic resonance device is configured to acquire magnetic resonance image data, particularly diagnostic magnetic resonance image data, from the object positioned within the imaging region. The object may be a patient, particularly a human or an animal.

The inventive magnetic resonance device may represent a closed bore scanner. A closed bore scanner may comprise a substantially cylindrical bore circumferentially enclosing the imaging region. The main magnet of the closed bore scanner may comprise one or more solenoidal superconducting coils circumferentially encompassing the imaging region along an axial direction or an axis of rotational symmetry of the cylindrical bore. The one or more superconducting coils may comprise a wire having a negligible electrical resistance at (or below) a superconducting temperature. A direction of a main magnetic field provided via the main magnet may be oriented substantially in parallel to a direction of access of the object to the imaging region and/or the axial direction of the cylindrical bore.

The magnetic resonance device comprises a magnet arrangement according to an embodiment described above.

The magnetic resonance device may comprise further components required for a proper operation of the magnetic resonance device. For example, the magnetic resonance device may comprise an outer vacuum chamber, a magnet support structure, a thermal shield, and a cryocooler. In certain embodiments, the magnetic resonance device comprises a cryogen vessel.

The cryogen vessel may be configured for storing or pre-serving a fluid, particularly a cryogen, at a predefined temperature level. Preferably, the fluid or cryogen exhibits a low boiling point, such as Argon, Nitrogen, Neon, Helium, or the like. The predefined temperature level may substantially correspond to a superconducting temperature of the main magnet.

The cryocooler may be configured to maintain the main magnet at a temperature level close to the superconducting temperature of the superconducting coils. The main magnet, the thermal shield, the magnet support structure, and/or the cryogen vessel may be thermally connected to the cryocooler via a solid thermal conductor, a convection loop and/or a heat pipe.

The inventive magnetic resonance device may represent a "dry" system comprising a minimum of cryogen or no cryogen at all. For example, the inventive magnetic resonance device may comprise one or more small cryogen vessels thermally connected to the main magnet via a solid thermal conductor. The one or more small cryogen vessels may contain a volume of less than 10 l, less than 5 l, or less than 1 l of cryogen. According to an embodiment of the inventive magnetic resonance device, a cryogen vessel is omitted. Thus, the main magnet may be cooled entirely via thermal conduction.

In an alternative embodiment, the magnetic resonance device represents a "wet" system. A "wet" system may include at least one cryogenic container with a volume of more than 10 l or more than 100 l. Preferably, the main magnet is accommodated within the cryogen vessel and cooled directly by the cryogen.

The magnetic resonance device shares the advantages of the inventive magnet arrangement according to an embodiment described above.

Further advantages and details of the present invention may be recognized from the embodiments described below as well as the drawings. The figures show:
Fig. 1 a schematic representation of an embodiment of an inventive magnetic resonance device,
Fig. 2 a schematic representation of an embodiment of a magnet arrangement,
Fig. 3 a schematic representation of an embodiment of a magnet arrangement,
Fig. 4 a schematic representation of an embodiment of an inventive magnet arrangement,
Fig. 5 a schematic representation of an embodiment of an inventive magnet arrangement,
Fig. 6 a schematic representation of an embodiment of an inventive magnet arrangement,
Fig. 7 a schematic representation of an embodiment of an inventive magnet arrangement,
Fig. 8 a schematic representation of an embodiment of an inventive magnet arrangement,
Fig. 9 a schematic representation of an embodiment of an inventive magnetic resonance device.

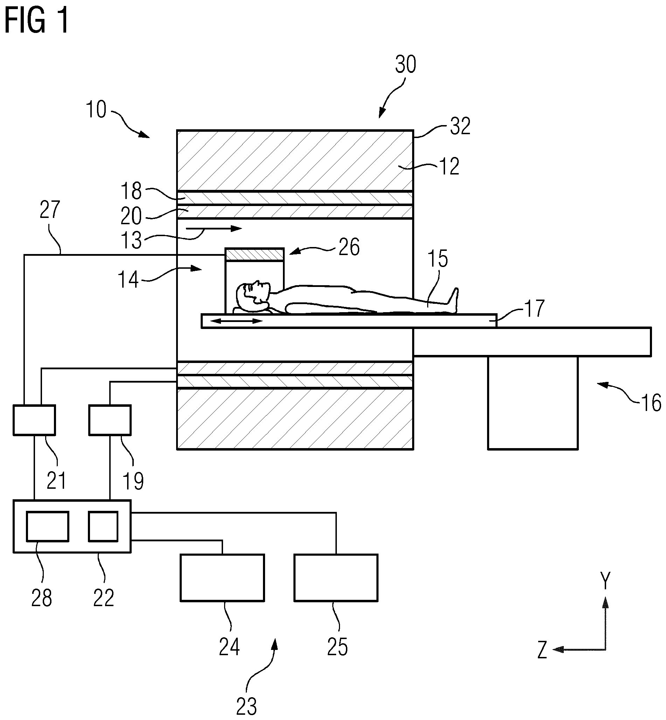

Fig. 1 shows an embodiment of a magnetic resonance device 10 according to the invention. In the illustrated example, the magnetic resonance device 10 comprises a static field magnet or main magnet 12 configured to provide a homogenous, static magnetic field 13 (B0 field) comprising an imaging volume (not shown). The static magnetic field 13 permeates an imaging region 14 configured to receive an imaging object, such as a patient 15. The imaging region 14 may correspond to a patient bore configured for accommodating a patient 15 during a magnetic resonance measurement. The imaging region 14 is encompassed by the main magnet 12 in a circumferential direction.

The magnetic resonance device 10 may comprise a patient positioning device 16 configured to transport the patient 15 into the imaging region 14. The patient support 16 may be configured to transport a diagnostically relevant body region of the patient 15 into the imaging volume or an isocentre of the magnetic resonance device 10. The main magnet 12 and other components of a field generation unit (not shown) of the magnetic resonance device 10 may be concealed in a housing 30.

The magnetic resonance device 10 may comprise a gradient system including one or more gradient coils 18. The one or more gradient coils may be configured to generate gradient magnetic fields in different, preferably orthogonally oriented, spatial directions. The gradient magnetic fields can be used for spatial encoding of magnetic resonance signals acquired during a magnetic resonance measurement. The one or more gradient coils 18 can be activated or controlled via an appropriate current signal provided by a gradient control unit 19.

The magnetic resonance device 10 may comprise an integrated radiofrequency antenna 20 (i. e. a body coil). The radiofrequency antenna 20 may be activated or controlled via a radiofrequency control unit 21. The radiofrequency control unit 21 may be configured to control the radiofrequency antenna 20 to generate a high frequency magnetic field and emit radiofrequency excitation pulses into the imaging region 14. The magnetic resonance device 10 may further comprise a local coil 26. The local coil 26 may be positioned on or in proximity to the diagnostically relevant region of the patient 15. The local coil 26 may be configured to emit radiofrequency excitation pulses into the patient 15 and/or receive magnetic resonance signals from the patient 15. It is conceivable, that the local coil 26 is controlled via the radiofrequency controller 21.

Preferably, the magnetic resonance device 10 comprises a control unit 22 configured to control the magnetic resonance device 10. The control unit 22 may comprise a processing unit 28 configured to process magnetic resonance signals and reconstruct magnetic resonance images. The processing unit 28 may also be configured to process input from a user of the magnetic resonance device 10 and/or provide an output to a user. For this purpose, the processing unit 28 and/or the control unit 22 can be connected to a display unit 24 and an input unit 25 via a suitable signal connection. For a preparation of a magnetic resonance measurement, preparatory information, such as imaging parameters or patient information, can be provided to the user via the display unit 24. The input unit 25 may be configured to receive information and/or imaging parameters from the user.

Of course, the magnetic resonance device 10 may comprise further components and/or functions which are common in magnetic resonance devices. The general operation of a magnetic resonance device 10 is known to those skilled in the art, so a more detailed description is omitted.

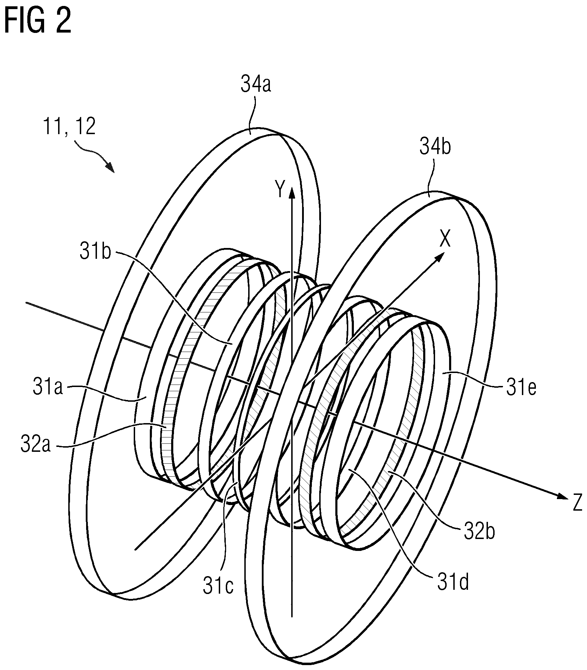

Fig. 2 shows an embodiment of an inventive magnet arrangement 11 without a ferromagnetic element 33. In the depicted example, the magnet arrangement 11 comprises five superconducting coils 31a, 31b, 31c, 31d, 31e (31a-e), and two reversed superconducting coils 32a and 32b (32a-b) constituting the main magnet 12. The magnet arrangement 11 also comprises two outer coils or shield coils 34a and 34b (34a-b) configured to shield the main magnet 12 from electromagnetic radiation. The superconducting coils 31a and 31e are arranged at axial ends of the main magnet 12 and constitute the end coils of the magnet arrangement 11.

In the depicted example, the reversed superconducting coil 32a is arranged between the two superconducting coils 31a and 31b. Likewise, the reversed superconducting coil 32b is arranged between the two superconducting coils 31d and 31e.

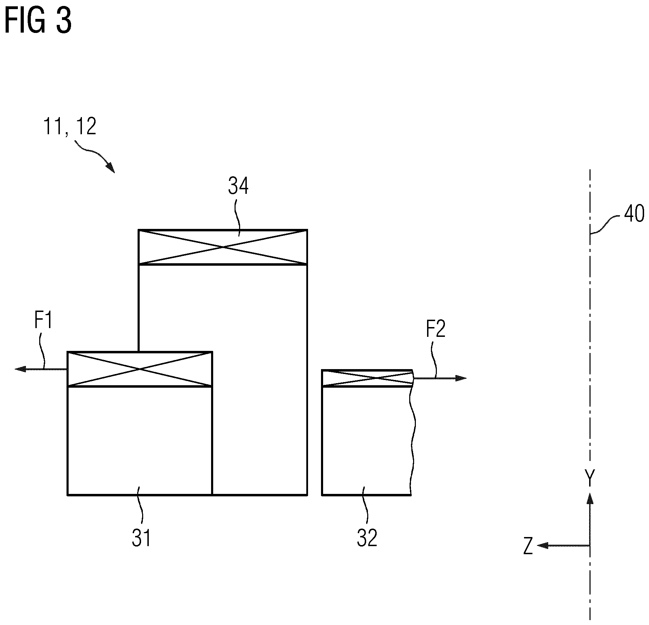

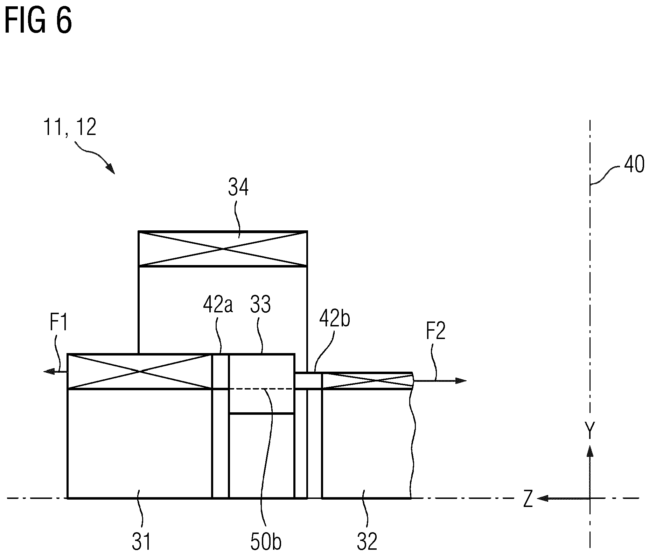

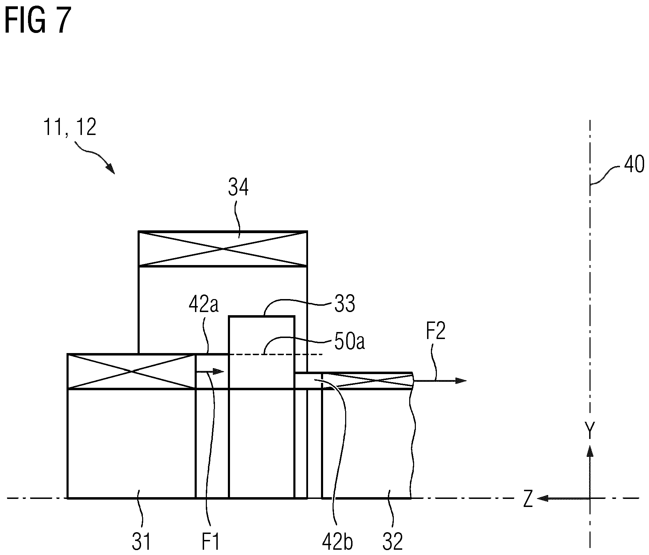

Fig. 3 shows a schematic representation of a cross-section through a section of the magnet arrangement 11 depicted in Fig. 2. In the depicted example, the superconducting coil 31 forms an end coil of the magnet arrangement 11. The reversed superconducting coil 32 is arranged adjacent to the end coil 31. It is conceivable that the end coil 31 and the reversed superconducting coil 32 are separated by a spacer 42 (not shown). For example, a spacer 42 may be arranged between the end coil 31 and the reversed superconducting coil 32 as shown in Figs. 6 or 7.

In absence of a ferromagnetic element 33, an electromagnetic force F2 acting on the reversed superconducting coil 32 may be directed towards a central symmetry plane 40 of the magnet arrangement 11, whereas an electromagnetic force F1 acting on the end coil 31 may be directed away from the central symmetry plane 40.

In certain embodiments, the electromagnetic force F2 may cause an equivalent weight of 19.1 t to act on the reversed superconducting coil 32. The electromagnetic force F1 may cause an equivalent weight of 11.4 t to act on the end coil 31. Thus, a magnet arrangement 11 according to Figs. 2 and 3 requires a dedicated support structure (not shown) configured to contain the electromagnetic forces F1 and F2 and maintain the main magnet 12, particularly the end coils 31a and 31e, in a predefined spatial arrangement relative to the reversed superconducting coils 32a-b, but also the superconducting coils 31b-d.

Depending on the design of the main magnet 12, such as the number of superconducting coils 31, the number of reversed superconducting coils 32, the diameter of the superconducting coils 31 and 32, the axial dimension of the superconducting coils 31 and 32, but also the number of windings of superconducting wire forming the superconducting coils 31 and 32, the electromagnetic force F1 may cause an equivalent weight of more than 11.4 t to act on the end coil 31. It is also conceivable that the equivalent weight is less than 11.4 t. In some embodiments, the electromagnetic force F1 acting on the end coil 31 may be directed towards the central symmetry plane 40 of the main magnet 11.

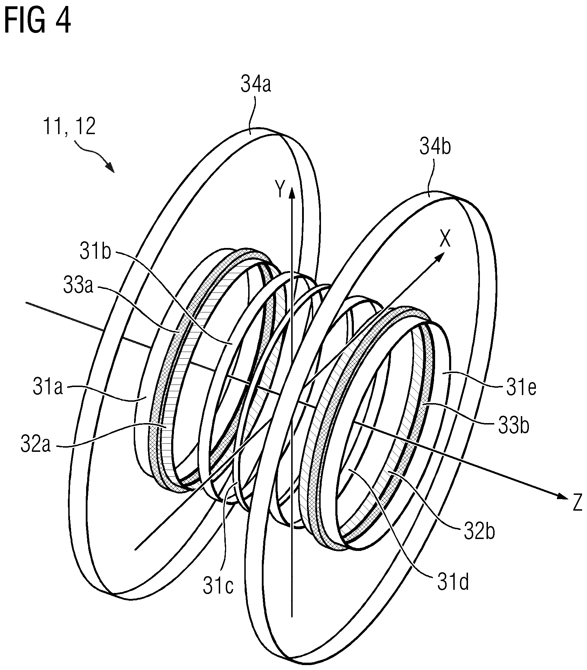

Fig. 4 shows an embodiment of an inventive magnet arrangement 11 comprising ferromagnetic elements 33a and 33b (33a-b). The ferromagnetic element 33a is arranged between the reversed superconducting coil 32a and the superconducting coil 31a. Likewise, the ferromagnetic element 33b is arranged between the reversed superconducting coil 32b and the superconducting coil 31e. The superconducting coils 31a and 31e may represent end coils of the magnet arrangement 11.

In some embodiments, the reversed superconducting coil 32a and the ferromagnetic element 33a may be arranged at a distance to an end coil. For example, the reversed superconducting coil 32a and the ferromagnetic element 33a may be arranged between the superconducting coils 31b and 31c. Likewise, the reversed superconducting coil 32b and the ferromagnetic element 33b may be arranged between the superconducting coils 31c and 31d.

The inventive magnet arrangement 11 may comprise more than five superconducting coils 31 and/or more than two reversed superconducting coils 32. However, it is conceivable that the inventive magnet arrangement 11 comprises less than 5 superconducting coils 31.

In a preferred embodiment of the inventive magnet arrangement 11, a ferromagnetic element 33 arranged adjacent to a reversed superconducting coil 32 confines a side or surface of the reversed superconducting coil 32 facing towards a closer axial end of the magnet arrangement 11.

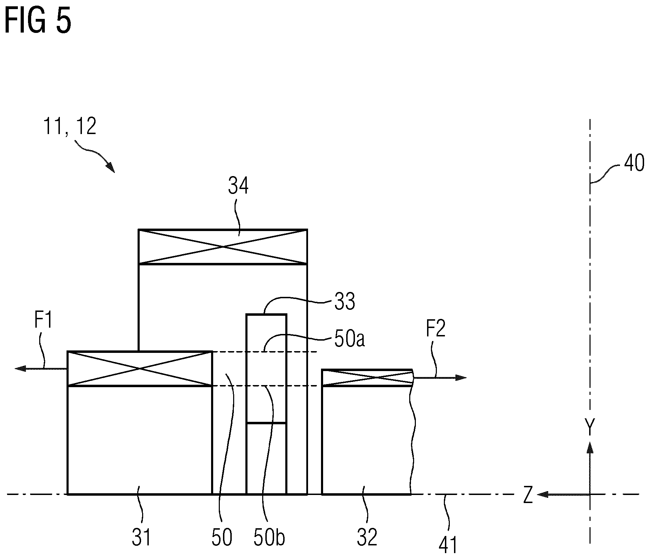

Fig. 5 shows a schematic representation of a cross section through a section of the inventive magnet arrangement 11 depicted in Fig. 4. In the depicted example, the ferromagnetic element 33 is arranged between the superconducting coil 31 and the reversed superconducting coil 32.

The ferromagnetic element 33 comprises the shape of a ring or a short cylinder. In the depicted example, the ferromagnetic element 33 is arranged coaxially to the main magnet 12 along a cylinder axis 41 of the main magnet 12. The ferromagnetic element 33 is arranged in such a way, that a projection 50 of an axial cross-sectional area of the superconducting coil 31 along the cylinder axis 41 of the main magnet 12 and a cross-sectional area of the ferromagnetic element 33 have a non-empty intersection.

In the embodiment shown in Fig. 5, a projection 50b of the inner circumference of the superconducting coil 31 along the cylinder axis 41 of the main magnet 12 intersects a section of the ferromagnetic element 33. Furthermore, a projection 50a of the outer circumference of the superconducting coil 31 along the cylinder axis 41 of the main magnet 12 intersects a section of the ferromagnetic element 33. In other words, the outer diameter of the ferromagnetic element 33 exceeds the outer diameter of the superconducting coil 31, whereas the inner diameter of the superconducting coil 31 exceeds the inner diameter of the ferromagnetic element 33.

The ferromagnetic element 33 preferably consists of iron or an iron alloy. Particularly, the ferromagnetic element 33 may be magnetically saturated in such a way, that it modifies inter-coil forces within the main magnet 12. In the embodiment depicted in Fig. 5, the ferromagnetic element 33 modifies inter-coil forces at an end section of the main magnet 12 in such a way that the electromagnetic force F1 is reduced by over 70 % in comparison to the embodiment depicted in Fig. 3.

For example, an equivalent weight acting on the superconducting coil 31 caused by the electromagnetic force F1 may amount to 5.4 t. In Fig. 5, the electromagnetic force F1 is still directed away from the central symmetry plane 40, but reduced to such an extent, that a magnet support structure (not shown) configured to maintain the superconducting coil 31 in a predefined spatial arrangement with respect to the other superconducting coils 31 of the main magnet 12 can be manufactured in a more cost-effective manner. Furthermore, the ferromagnetic element 33 modifies inter-coil forces within the main magnet 12 in such a way that an equivalent weight acting on the reversed superconducting coil 32 caused by the electromagnetic force F2 increases to about 17.3 t.

Fig. 6 shows a further embodiment of the inventive magnet arrangement 11. In the depicted example, the ferromagnetic element 33 is configured to modify an inter-coil force within the main magnet 12 in such a way that the electromagnetic force F1 acting on the superconducting coil 31 is substantially cancelled out or reduced to zero.

The inner circumference or inner diameter of the superconducting coil 31 exceeds the inner circumference or inner diameter of the ferromagnetic element 33 in such a way that a projection 50b of the inner circumference of the superconducting coil 31 along the cylinder axis 41 of the main magnet 12 intersects a section of the ferromagnetic element 33.

In the embodiment depicted in Fig. 6, a spacer 42a is arranged between the superconducting coil 31 and the ferromagnetic element 33, and a spacer 42b is arranged between the ferromagnetic element 33 and the reversed superconducting coil 32. The spacers 42a and 42b (42a-b) may comprise or consist of a rigid material, such as a metal, a metal alloy, a plastic and/or a composite material. Preferably, the material of the spacers 42a-b is configured to withstand the (compressive) inter-coil forces within the main magnet 12.

According to an embodiment, the spacers 42a-b are glued or bonded to the superconducting coil 31, the ferromagnetic element 33, and the reversed superconducting coil 32 to form an integrally bonded or cohesive structure. Preferably, an adhesive providing a material bond between the spacers 42a-b, the ferromagnetic element 33, the superconducting coil 31 and/or the reversed coil 33 is thermally conductive.

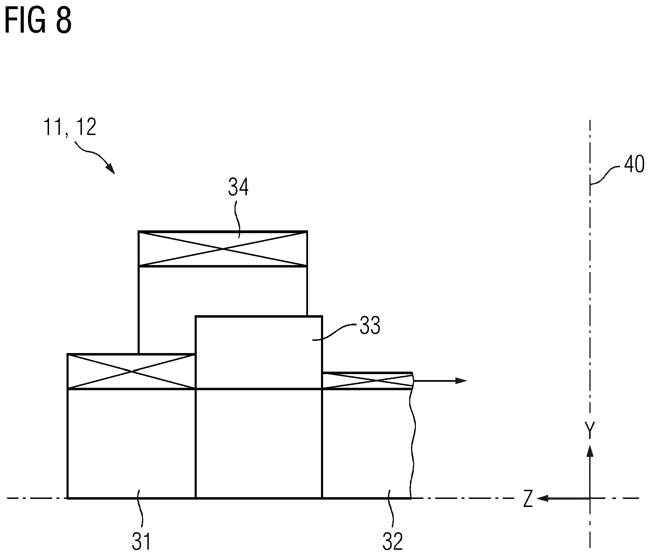

In an alternative embodiment, the ferromagnetic element 33 may be arranged directly adjacent to the superconducting coil 31 and/or the reversed superconducting coil 32 (see Fig. 8). For example, the ferromagnetic element 33 may directly contact the superconducting coil 31 and/or the reversed superconducting coil 32. Particularly, an axial face or side of the ferromagnetic element 33 may be glued or bonded to an axial face of the superconducting coil 31 and/or an axial face of the reversed superconducting coil 32.

Fig. 7 shows a further embodiment of the inventive magnet arrangement 11. In the depicted example, the ferromagnetic element 33 is configured to modify an inter-coil force within the main magnet 12 in such a way that the electromagnetic force F1 acting on the superconducting coil 31 is inverted in comparison to the embodiment depicted in Fig. 3. Particularly, the ferromagnetic element 33 is configured to modify the inter-coil force within the main magnet 12 in such a way that an inwardly directed force F1 is acting on the superconducting coil 31. For example, an equivalent weight acting on the superconducting coil 31 due electromagnetic force F1 may amount to about 5.5 t.

In the present example, the outer circumference of the ferromagnetic element 33 exceeds the outer circumference of the superconducting coil 31 in such a way that a projection 50a of the outer circumference of the superconducting coil 31 along the cylinder axis 41 of the main magnet 12 intersects a section of the ferromagnetic element 33. The inner circumference of the ferromagnetic element 33 may correspond to an inner circumference of the superconducting coil 31 and/or the reversed superconducting coil 32. It is also conceivable, that the inner circumference of the ferromagnetic element 33 exceeds the inner circumference of the superconducting coil 31 and/or an inner circumference of the reversed superconducting coil 32.

In the embodiment of the inventive magnet arrangement 11 depicted in Fig. 8, the ferromagnetic element 33 is directly mechanically connected to the superconducting coil 31 and the reversed superconducting coil 32. Particularly, a first axial face of the ferromagnetic element 33 is in contact with an axial face of the superconducting coil 31 and a second axial face of the ferromagnetic element 33 is in contact with an axial face of the reversed superconducting coil 32.

The ferromagnetic element 33 may comprise an electrically insulating layer which is configured to prevent electrical current from passing through the ferromagnetic element 33. For example, the first axial face and/or the second axial face of the ferromagnetic element 33 may be clad, laminated, coated, or otherwise provided with an electrically insulating material. Preferably, the electrically insulating material is thermally conductive. According to an embodiment, an adhesive providing a material bond between the ferromagnetic element 33 and the superconducting coil 31, but also between the ferromagnetic element 33 and the reversed coil 33, is thermally conductive but electrically insulating.

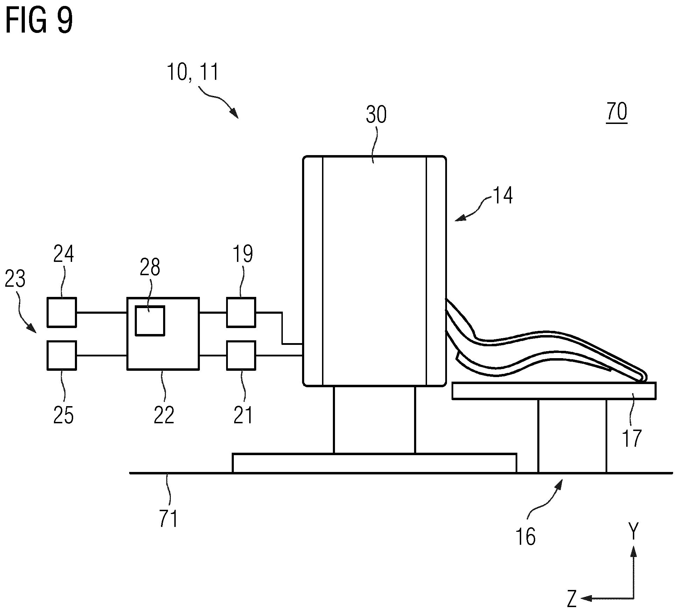

Fig. 9 shows a further embodiment of a magnetic resonance device 10 according to the invention. The functions and components of the magnetic resonance device 10 shown in Fig. 9 may correspond to the functions and components of the magnetic resonance device 10 depicted in Fig. 1.

For example, the magnetic resonance device 10 may be configured to perform a magnetic resonance examination of a jaw region and/or an eye region of a patient 15. The inventive magnetic resonance device 10 may also be configured to perform cardiac imaging, mammography imaging, neurological imaging, urological imaging, orthopaedic imaging, prostate imaging, or imaging of other parts of the patient's body. In particular, the magnetic resonance device 10 may represent a dedicated scanner designed to perform magnetic resonance imaging of a jaw region and/or head region of a standing or sitting patient 15. The magnetic resonance device 10 may be significantly shorter along the Z-axis in comparison to conventional cylindrical (or closed-bore) magnetic resonance devices. Thus, the inventive magnetic resonance device 10 may be favourably transported to and/or installed in smaller medical facilities or practises via conventional or standardized access routes without interrupting operations due to construction sites or renovation work.

In the example shown in Fig. 9, the magnetic resonance device 10 is supported via a mount 31. The mount 31 is configured to carry the magnet arrangement 11 and maintain the magnet arrangement 11 at a predetermined distance from a floor 71 of an examination room 70. It is conceivable that the mount 31 has a positioning unit (not shown) that is designed to position and/or align the magnet arrangement 11 relative to a diagnostically relevant body region of the patient 15. For example, the positioning unit may include a swivel joint that is designed to rotate the magnet arrangement 11 along a direction of rotation. The mount 31 may also comprise a telescopic guide and/or a rail system configured to adjust a spatial position of the magnet arrangement 11 along a Y-direction and/or a Z-direction.

It is conceivable that the magnetic resonance device 10 includes a patient positioning device 16 and/or a patient table 17 as shown in Fig. 1. The patient positioning device 16 and/or patient table 17 may be configured to position a diagnostically relevant body region of patient 15 in the imaging region 14.

As an alternative to the embodiment shown in Fig. 9, the mount 31 may be configured to attach the magnetic resonance device 10 or the magnet arrangement 11 to a wall and/or ceiling of an examination room 70.

The embodiments described herein are to be recognized as examples. It is to be understood that individual embodiments may be extended by or combined with features of other embodiments if not stated otherwise. The embodiments depicted in the Figs. 1 to 9 are representations that do not necessarily have to be to scale.

## Claims

1. A magnet arrangement (11) for a magnetic resonance imaging device (10), comprising a main magnet (12) including a plurality of superconducting coils (31), a reversed superconducting coil (32), and a ferromagnetic element (33), wherein the reversed superconducting coil (32) is arranged between two superconducting coils (31) of the plurality of superconducting coils (31), and wherein the ferromagnetic element (33) is arranged between the reversed superconducting coil (32) and a superconducting coil (31).

2. The magnet arrangement (11) according to claim 1, wherein the superconducting coil (31) is an end coil of the main magnet (12).

3. The magnet arrangement (11) according to one of the preceding claims, wherein the ferromagnetic element (33) is arranged directly adjacent to the reversed superconducting coil (32) and/or the superconducting coil (31).

4. The magnet arrangement (11) according to claim 1 or 2, wherein the main magnet (12) comprises at least one spacer (42) arranged between the ferromagnetic element (33) and the superconducting coil (31) and/or between the ferromagnetic element (33) and the reversed superconducting coil (32).

5. The magnet arrangement (11) according to one of the preceding claims, wherein the ferromagnetic element (33) comprises the shape of a ring, a tube, a hollow cylinder, or a hollow prism.

6. The magnet arrangement (11) according to claim 5, wherein the main magnet (12) comprises a cylindrical shape, and wherein the ferromagnetic element (33) is arranged coaxially to the main magnet (12).

7. The magnet arrangement (11) according to claim 6, wherein a projection (50) of an axial cross-sectional area of the superconducting coil (31) along a cylinder axis (41) of the main magnet (12) and a cross-sectional area of the ferromagnetic element (33) have a non-empty intersection.

8. The magnet arrangement (11) according to one of the preceding claims, wherein the ferromagnetic element (33) comprises or consists of a high permeability material.

9. The magnet arrangement (11) according to one of the preceding claims, wherein the ferromagnetic element (33) is attached to the reversed superconducting coil (32) and the superconducting coil (31).

10. The magnet arrangement (11) according to one of the preceding claims, wherein the superconducting coil (31) represents an end coil and wherein the ferromagnetic element (33) is configured to modify an inter-coil force within the main magnet (12) in such a way that an outwardly directed force, which would act on the end coil of the main magnet (12) in absence of the ferromagnetic element (33), is reduced by at least 30 %, at least 40 %, at least 50 %, or at least 60 %.

11. The magnet arrangement (11) according to claim 10, wherein the ferromagnetic element (33) is configured to modify the inter-coil force within the main magnet (12) in such a way that an inwardly directed force is acting on the superconducting coil (31).

12. The magnet arrangement (11) according to one of the claims 1 to 9, wherein the superconducting coil (31) represents an end coil and wherein the ferromagnetic element (33) is configured to modify an inter-coil force within the main magnet (12) in such a way that an inwardly directed force, which would act on the end coil in absence of the ferromagnetic element (33), is increased by at least 10 %, at least 20 %, or at least 30 %.

13. The magnet arrangement (11) according to one of the preceding claims, comprising a first ferromagnetic element (33a) and a second ferromagnetic element (33b), wherein the first ferromagnetic element (33a) is arranged between a first reversed superconducting coil (32) and a first superconducting coil (31a), and the second ferromagnetic element (33b) is arranged between a second reversed superconducting coil (32b) and a second superconducting coil (31e).

14. A magnetic resonance device (10) for acquiring magnetic resonance data of an object positioned within an imaging region (14) of the magnetic resonance device (10), comprising a magnet arrangement (11) according to one of the preceding claims.
